# EUROPEAN PATENT APPLICATION

(11) **EP 3 029 025 A1**
(43) Date of publication of application: **08.06.2016**
(21) Application number: 14196527.7
(22) Date of filing: 05.12.2014
(51) Int. Cl.: C07C 249/12, A01N 37/50, A01N 37/52, A01P 3/00

(54) **Method for combating soybean rust comprising treating soybean with (2e)-2-methoxyimino-2 [2 [[(e)-[(2e)-2-alkoxyimino-1-methyl-alk-3-enylidene]amino]oxymethyl]phenyl]-n-methyl-acetamides**

(71) Applicant: BASF SE, 67056 Ludwigshafen am Rhein (DE)
(72) Inventor: Grammenos, Wassilios, 67071 Ludwigshafen (DE); Escribano Cuesta, Ana, 68161 Mannheim (DE); Grote, Thomas, 67157 Wachenheim (DE); Haden, Egon, 67346 Speyer (DE); Craig, Ian Robert, 67063 Ludwigshafen (DE); Brahm, Lutz, 67551 Worms (DE)
(74) Representative: BASF IP Association

(57) **Abstract**

The present invention relates to the use of for combating phytopathogenic soybean rust, and to seeds coated with (2*E*)-2-methoxyimino-2-[2-[[(*E*)-[(2*E*)-2-alkoxyimino-1-methyl-alk-3-enylidene]amino]oxymethyl]phenyl]-*N*-methyl-acetamides. The invention also relates to methods for combating soybean rust (a disease caused by *Phakopsora pachyrhizi* and P. *meibomiae),* comprising treating soybean plants or seeds to be protected against attack by the fungi *Phakopsora pachyrhizi* and P. *meibomiae.* The invention also relates to novel (2*E*)-2-methoxyimino-2-[2-[[(*E*)-[(2*E*)-2-alkoxyimino-1-methyl-alk-3-enylidene]amino]oxymethyl]phenyl]N-methyl-acetamide compounds.

## Description

The present invention relates to the use of (2*E*)-2-methoxyimino-2-[2-[[(*E*)-[(2*E*)-2-alkoxy-imino-1-methyl-alk-3-enylidene]amino]oxymethyl]phenyl]-*N*-methyl-acetamides for combating phytopathogenic soybean rust, and to seeds coated with (2*E*)-2-methoxyimino-2-[2-[[(*E*)-[(2*E*)-2-alkoxyimino-1-methyl-alk-3-enylidene]amino]oxymethyl]phenyl]-*N*-methyl-acetamides. The invention also relates to methods for combating soybean rust (a disease caused by *Phakopsora pachyrhizi* and *P. meibomiae),* comprising treating soybean plants or seeds to be protected against attack by the fungi *Phakopsora pachyrhizi* and *P. meibomiae.* The invention also relates to novel (2*E*)-2-methoxyimino-2-[2-[[(*E*)-[(2*E*)-2-alkoxyimino-1-methyl-alk-3-enylidene]amino]oxymethyl]phenyl]-*N*-methyl-acetamide compounds.

Soybean rust is a serious disease in plants, particular in soybean plants, that is caused by the plant-pathogenic basidiomycete fungi *Phakopsora pachyrhizi* and *P. meibomiae. P. pachyrhizi* originated in Asia, whereby it is also called the causal agent of Asian soybean rust, whereas the less aggressive species *P. meiborniae* originated in Latin America. In the New World, *P*. *pachyrhizi* was first reported in the 1990s to have spread to Hawaii and, since 2001, it has been found in South America (Mol. Plant Pathol. 11(2), 169-177, 2010). Soybean rust is spread by windblown spores and has caused siginificant crop losses in many soybean-growing regions of the world (http://www.ppdl.purdue.edu/ppdl/soybean_rust.html).

WO 95/021154 generally mentions phenyl acetic acid derivatives of the generic formula: 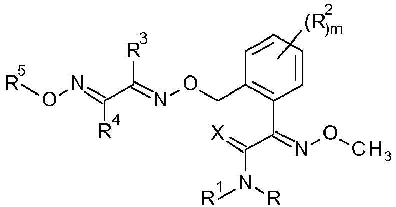 and their use for fungicidal use against certain plant-pathogenic fungi. However, WO 95/021154 does not disclose any action against soybean rust nor does this document mention the specific (2*E*)-2-methoxyimino-2-[2-[[(*E*)-[(2*E*)-2-alkoxyimino-1-methyl-alk-3-enylidene]amino]oxymethyl]-phenyl]-*N*-methyl-acetamides according to this invention.

The use of certain strobilurin-like compounds for combating soybean rust, is known inter alia from WO 11/095134. The compounds according to the present invention differ from those described in WO 11/095134 inter alia by the specific double oxim-ether side chain and the phenyl-2-methoxyimino-*N*-methyl acetmaide pharmacophor as defined herein.

In many cases, in particular at low application rates, the fungicidal activity of the known fungicidal compounds especially against soybean rust is unsatisfactory. Based on this, it was an object of the present invention to provide a use for control of soybean rust with compounds having improved activity against soybean rust caused by the fungi *Phakopsora pachyrhizi* and *P*. *meibomiae.*

This object is achieved by using (2*E*)-2-methoxyimino-2-[2-[[(*E*)-[(2*E*)-2-aikoxyimino-1-methyl-alk-3-enylidene]amino]oxymethyl]phenyl]-*N*-methyl-acetamides against soybean rust disease caused by *Phakopsora pachyrhizi* and *P. meibomiae.*

Accordingly, the present invention relates to the use of compound of formula I: 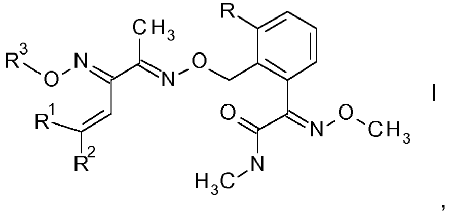 wherein
- R: is hydrogen, halogen or methyl;
- R¹,R²: independently of each other are selected from hydrogen, C₁-C₄-alkyl and C₁-C₄-haloalkyl;
or R¹ and R² together with the carbon atom linking them form a C₃-C₆-cycloalkyl;
- R³: is C₁-C₄-alkyl;
for combating soybean rust.

The term "compounds I" refers to compounds of formula I.

A further embodiment relates to the use of compounds I for combating soybean rust on soybean plants and on the plant propagation material, such as seeds, and the crop material of these plants. Soybean rust is cause by two fungal pathogens called *Phakopsora pachyrhizi* and *P. meibomiae.*

Consequently, a further embodiment relates to the use of compounds I for combating *Phakopsora pachyrhizi* and/or *P*. *meibomiae* on soybean plants and on the plant propagation material, such as seeds, and the crop material of these plants. A more preferred embodiment the use of compounds I for combating *Phakopsora pachyrhizi* on soybean plants and on the plant propagation material, such as seeds, and the crop material of these plants relates

Accordingly, the present invention relates to the method for combating soybean rust (*Phakopsora pachyrhizi* and/or *P*. *meibomiae*), comprising:
treating the soybean plants or soybean plant propagation material to be protected against attack by *Phakopsora pachyrhizi* and/or *P*. *meibomiae* with an effective amount of at least one compound I, or a composition comprising such compound I.

Treatment against soybean rust can be preventive or curative.

Preferably treatment of soybean plants against soybean rust shall be preventive. Preventive treatment shall be performed when the soybean plants are at risk of infection latest shartly after the first symptoms are visible. According to one embodiment, the first treating of the soybean plants shall take place at the vegetative growth stages V3 to V4 (meaning 4 to 4 fully expanded trifoliate leaves) onwards to the reproductive growth stage R2 (full bloom), more preferably place at the vegetative growth stages V6 to V8 (meaning 6 to 8 fully expanded trifoliate leaves) onwards to the reproductive growth stage R1 (beginning bloom). Depending on the disease pressure, two to four and under extreme conditions up to five applications may be necessary at application intervals of 10 to 21 days.

When employed as foliar spray against soybean rust, the amounts of the compounds I applied are, depending on the specific compound used and on the disease pressure, from 5 g to 500 g per ha, preferably from 10 to 200 per ha, more preferably from 25 to 125 g per ha, and in particular from 30 to 80 g per ha.

The preparation of one compound of formula I, (2*E*)-2-methoxyimino-2-[2-[[(*E*)-[(2*E*)-2-methoxyimino-1,4-dimethyl-pent-3-enylidene]amino]oxymethyl]phenyl]-*N*-methyl-acetamide is disclosed in WO 00/0311023. However, it is not mentioned therein that this or other compounds disclosed therein can be used for combating soybean rust.

The compounds according to the present invention differ from those described in the abovementioned publication that (2*E*)-2-methoxyimino-2-[2-[[(*E*)-[(2*E*)-2-methoxyimino-1,4-dimethyl-pent-3-enylidene]amino]oxymethyl]phenyl]-*N*-methyl-acetamide is excluded.

Therefore, according to a further aspect, the invention provides compounds of formula I which are represented by formula I 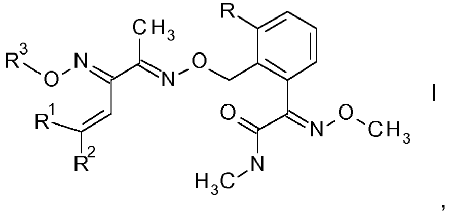 wherein
- R: is hydrogen, halogen or methyl;
- R¹,R²: independently of each other are selected from hydrogen, C₁-C₄-alkyl and C₁-C₄-haloalkyl;
or R¹ and R² together with the carbon atom linking them form a C₃-C₆-cycloalkyl;
- R³: is C₁-C₄-alkyl;
except for (2*E*)-2-methoxyimino-2-[2-[[(*E*)-[(2*E*)-2-methoxyimino-1,4-dimethyl-pent-3-en-ylidene]amino]oxymethyl]phenyl]-*N*-methyl-acetamide.

The compounds I can be present in atropisomers arising from restricted rotation about a single bond of asymmetric groups. They also form part of the subject matter of the present invention.

Depending on the substitution pattern, the compounds I may have one or more centers of chirality, in which case they are present as pure enantiomers or pure diastereomers or as enantiomer or diastereomer mixtures. Both, the pure enantiomers or diastereomers and their mixtures are subject matter of the present invention.

In the definitions of the symbols given in the formulae herein, collective terms were used which are generally representative for the following substituents:
halogen: fluorine, chlorine, bromine and iodine;
C₁-C₄-alkyl: saturated straight-chain or branched hydrocarbon groups having 1 to 4 carbon atoms, such as methyl, ethyl, propyl, 1-methylethyl, butyl, 1-methylpropyl, 2-methylpropyl, 1,1-dimethylethyl, pentyl, 1-methylbutyl, 2-methylbutyl, 3-methylbutyl, 2,2-dimethylpropyl, 1-ethylpropyl, 1,1-dimethylpropyl, 1,2-dimethylpropyl;
C₁-C₄-haloalkyl: straight-chain or branched alkyl groups having 1 to 4 carbon atoms (as mentioned above), where some or all of the hydrogen atoms in these groups may be replaced by halogen atoms as mentioned above: in particular halomethyl, such as chloromethyl, bromomethyl, dichloromethyl, trichloromethyl, fluoromethyl, difluoromethyl, trifluoromethyl, chlorofluoro-methyl, dichlorofluoromethyl, chlorodifluoromethyl; and haloethyl such as 1-chloroethyl, 1-bro-moethyl, 1-fluoroethyl, 2-fluoroethyl, 2,2-difluoroethyl, 2,2,2-trifluoroethyl, 2-chloro-2-fluoroethyl, 2-chloro-2,2-difluoroethyl, 2,2-dichloro-2-fluoroethyl, 2,2,2-trichloroethyl, pentafluoroethyl or 1,1,1-trifluoroprop-2-yl;
C₃-C₆-cycloalkyl: monocyclic saturated hydrocarbon groups having 3 to 6 carbon ring members, such as cyclopropyl, cyclobutyl, cyclopentyl or cyclohexyl;

Preference is given to those compounds I and where applicable also to the compounds of all sub-formulae provided herein and in particular to the use and/or method of treatment as defined above of those compounds, wherein the substituents and variables (such as R, R¹, R², and R³) have independently of each other or more preferably in combination the following meanings:
According to one embodiment of the invention, R is halogen, more preferably Cl or F, in particular Cl.
According to a further embodiment, R is methyl.
According to a further embodiment, R is hydrogen.
According to a further embodiment, R¹ and R² both are hydrogen.
According, to a further embodiment, R² is C₁-C₄-alkyl, more preferably methyl or ethyl.
According, to a further embodiment, R¹ and R² both are C₁-C₄-alkyl, more preferably independently from each other methyl or ethyl and in particular both are methyl.
According to a further embodiment, R¹ and R² together with the carbon atom linking them form a C₃-C₆-cycloalkyl, more preferably cyclopropyl or cyclopentyl; in particular cyclopentyl.
According to a further embodiment, R³ is methyl or ethyl; in particular methyl.
According to a further embodiment, R is hydrogen, and R³ is methyl or ethyl; in particular methyl.
According to a further embodiment, R is hydrogen, and R³ is methyl or ethyl; in particular methyl.

Particular preference is given to the use and method of compound I.1 which is of the following formula: 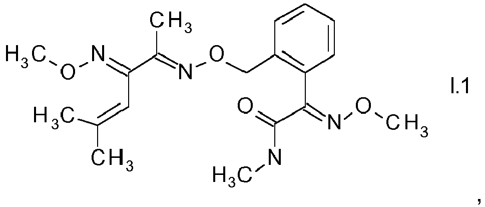 which according to IUPAC nomenclature is (2*E*)-2-methoxyimino-2-[2-[[(*E*)-[(2*E*)-2-methoxy-imino-1,4-dimethyl-pent-3-enylidene]amino]oxymethyl]phenyl]-*N*-methyl-acetamide.

Particular preference is given to method for combating soybean rust as defined herein wherein compound I is compound I.1.

Compounds I may be obtained as described in WO 00/0311023.

The term "plant propagation material" is to be understood to denote all the generative parts of the plant such as seeds and vegetative plant material such as cuttings and tubers (e. g. potatoes), which can be used for the multiplication of the plant. This includes seeds, roots, fruits, tubers, bulbs, rhizomes, shoots, sprouts and other parts of plants, including seedlings and young plants, which are to be transplanted after germination or after emergence from soil. These young plants may also be protected before transplantation by a total or partial treatment by immersion or pouring.

Preferably, treatment of plant propagation materials with compound I and compositions thereof, respectively, is used for controlling soyben rust on soybeans.

The term "soybean plants" is to be understood as including soybean plants which have been modified by breeding, mutagenesis or genetic engineering including but not limiting to agricultural biotech products on the market or in development (cf. http://www.bio.org/speeches/pubs/ er/agri_products.asp). Genetically modified plants are plants, which genetic material has been so modified by the use of recombinant DNA techniques that under natural circumstances cannot readily be obtained by cross breeding, mutations or natural recombination. Typically, one or more genes have been integrated into the genetic material of a genetically modified plant in order to improve certain properties of the plant. Such genetic modifications also include but are not limited to targeted post-translational modification of protein(s), oligo- or polypeptides e. g. by glycosylation or polymer additions such as prenylated, acetylated or farnesylated moieties or PEG moieties.

Plants that have been modified by breeding, mutagenesis or genetic engineering, e. g. have been rendered tolerant to applications of specific classes of herbicides, such as auxin herbicides such as dicamba or 2,4-D; bleacher herbicides such as hydroxylphenylpyruvate dioxygenase (HPPD) inhibitors or phytoene desaturase (PDS) inhibittors; acetolactate synthase (ALS) inhibitors such as sulfonyl ureas or imidazolinones; enolpyruvylshikimate-3-phosphate synthase (EPSPS) inhibitors, such as glyphosate; glutamine synthetase (GS) inhibitors such as glufosinate; protoporphyrinogen-IX oxidase inhibitors; lipid biosynthesis inhibitors such as acetyl CoA carboxylase (ACCase) inhibitors; or oxynil (i. e. bromoxynil or ioxynil) herbicides as a result of conventional methods of breeding or genetic engineering. Furthermore, plants have been made resistant to multiple classes of herbicides through multiple genetic modifications, such as resistance to both glyphosate and glufosinate or to both glyphosate and a herbicide from another class such as ALS inhibitors, HPPD inhibitors, auxin herbicides, or ACCase inhibitors. These herbicide resistance technologies are e. g. described in Pest Managem. Sci. 61, 2005, 246; 61, 2005, 258; 61, 2005, 277; 61, 2005, 269; 61, 2005, 286; 64, 2008, 326; 64, 2008, 332; Weed Sci. 57, 2009, 108; Austral. J. Agricult. Res. 58, 2007, 708; Science 316, 2007, 1185; and references quoted therein. Several cultivated plants have been rendered tolerant to herbicides by conventional methods of breeding (mutagenesis), e. g. Clearfield^{®} being tolerant to imidazolinones, e. g. imazamox. Genetic engineering methods have been used to render cultivated soybean plants tolerant to herbicides such as dicamba, glyphosate, imidazolinone and glufosinate or a combination of any of them, some of which are commercially available under the trade names RoundupReady^{®} (glyphosate-tolerant, Monsanto, U.S.A.), Cultivance^{®} (imidazolinone tolerant, BASF SE, Germany) and LibertyLink^{®} (glufosinate-tolerant, Bayer CropScience, Germany).

Furthermore, plants are also covered that are by the use of recombinant DNA techniques capable to synthesize one or more insecticidal proteins, especially those known from the bacterial genus *Bacillus,* particularly from *Bacillus thuringiensis,* such as δ-endotoxins, e. g. CryIA(b), CryIA(c), CryIF, CryIF(a2), CryIIA(b), CryIIIA, CryIIIB(b1) or Cry9c; vegetative insecticidal proteins (VIP), e. g. VIP1, VIP2, VIP3 or VIP3A; insecticidal proteins of bacteria colonizing nematodes, e. g. *Photorhabdus* spp. or *Xenorhabdus* spp.; toxins produced by animals, such as scorpion toxins, arachnid toxins, wasp toxins, or other insect-specific neurotoxins; toxins produced by fungi, such Streptomycetes toxins, plant lectins, such as pea or barley lectins; agglutinins; proteinase inhibitors, such as trypsin inhibitors, serine protease inhibitors, patatin, cystatin or papain inhibitors; ribosome-inactivating proteins (RIP), such as ricin, maize-RIP, abrin, luffin, saporin or bryodin; steroid metabolism enzymes, such as 3-hydroxysteroid oxidase, ecdysteroid-IDP-glycosyl-transferase, cholesterol oxidases, ecdysone inhibitors or HMG-CoA-reductase; ion channel blockers, such as blockers of sodium or calcium channels; juvenile hormone esterase; diuretic hormone receptors (helicokinin receptors); stilben synthase, bibenzyl synthase, chitinases or glucanases. In the context of the present invention these insecticidal proteins or toxins are to be understood expressly also as pre-toxins, hybrid proteins, truncated or otherwise modified proteins. Hybrid proteins are characterized by a new combination of protein domains, (see, e. g. WO 02/015701).

Furthermore, plants are also covered that are by the use of recombinant DNA techniques capable to synthesize one or more proteins to increase the resistance or tolerance of those plants to bacterial, viral or fungal pathogens. Examples of such proteins are the so-called "pathogenesis-related proteins" (PR proteins, see, e. g. EP-A 392 225), plant disease resistance genes or T4-lysozym. The methods for producing such genetically modified plants are generally known to the person skilled in the art and are described, e. g. in the publications mentioned above.

Furthermore, plants are also covered that are by the use of recombinant DNA techniques capable to synthesize one or more proteins to increase the productivity (e. g. bio mass production, grain yield, starch content, oil content or protein content), tolerance to drought, salinity or other growth-limiting environmental factors or tolerance to pests and fungal, bacterial or viral pathogens of those plants.

According to a further embodiment, compound I is in addition suitable for controlling on soybean plants the phytopathogenic fungi selected from *Cercospora sojina* and *C. kikuchii, Colleotrichum gloeosporioides, Corynespora cassiicola* (leaf spots), *Dematophora* (teleomorph: *Rosellinia) necatrix* (root and stem rot), *Diaporthe* spp., e. g. *D. phaseolorum* (damping off), *Fusarium solani, Microsphaera diffusa* (powdery mildew), *Peronospora manshurica* (downy mildew), *Phakopsora pachyrhizi* and *P. meibomiae* (soybean rust), *Phytophthora megasperma* (syn. *P. sojae*), *Rhizoctonia solani* (root and stem rot), *Septoria glycines* (brown spot) and *Thielaviopsis* spp. (black root rot).

The compound I and compositions thereof, resepectively, may be used for improving the health of a plant. The invention also relates to a method for improving plant health by treating a plant, its propagation material and/or the locus where the plant is growing or is to grow with an effective amount of compounds I and compositions thereof, respectively. Preferably, the invention relates to the use for improving the health of a soybean plant.

The term "plant health" is to be understood to denote a condition of the plant and/or its products which is determined by several indicators alone or in combination with each other such as yield (e. g. increased biomass and/or increased content of valuable ingredients), plant vigor (e. g. improved plant growth and/or greener leaves ("greening effect")), quality (e. g. improved content or composition of certain ingredients) and tolerance to abiotic and/or biotic stress.The above identified indicators for the health condition of a plant may be interdependent or may result from each other.

The compound of formula I can be present in different crystal modifications whose biological activity may differ. They are likewise subject matter of the present invention.

The compound I is employed as such or in form of compositions by treating the fungi or the plants, plant propagation materials, such as seeds, or soil, with a fungicidally effective amount of the active substances. The application can be carried out both before and after the infection of the soybean plants, plant propagation materials, such as seeds, or soil.

Plant propagation materials may be treated with compounds I as such or a composition comprising at least one compound I prophylactically either at or before planting or transplanting.

The invention also relates to agrochemical compositions comprising an auxiliary and compound I according to the invention.

An agrochemical composition comprises a fungicidally effective amount of compound I. The term "effective amount" denotes an amount of the composition or of the compounds I, which is sufficient for controlling harmful fungi on soybean plants or in the protection of materials and which does not result in a substantial damage to the treated plants. Such an amount can vary in a broad range and is dependent on various factors, such as the fungal species to be controlled and the climatic conditions.

The compound I can be converted into customary types of agrochemical compositions, e. g. solutions, emulsions, suspensions, dusts, powders, pastes, granules, pressings, capsules, and mixtures thereof. Examples for composition types are suspensions (e. g. SC, OD, FS), emulsifiable concentrates (e. g. EC), emulsions (e. g. EW, EO, ES, ME), capsules (e. g. CS, ZC), pastes, pastilles, wettable powders or dusts (e. g. WP, SP, WS, DP, DS), pressings (e. g. BR, TB, DT), granules (e. g. WG, SG, GR, FG, GG, MG), insecticidal articles (e. g. LN), as well as gel formulations for the treatment of plant propagation materials such as seeds (e. g. GF). These and further compositions types are defined in the "Catalogue of pesticide formulation types and international coding system", Technical Monograph No. 2, 6th Ed. May 2008, CropLife International.

The compositions are prepared in a known manner, such as described by Mollet and Grubemann, Formulation technology, Wiley VCH, Weinheim, 2001; or Knowles, New developments in crop protection product formulation, Agrow Reports DS243, T&F Informa, London, 2005.

Suitable auxiliaries are solvents, liquid carriers, solid carriers or fillers, surfactants, dispersants, emulsifiers, wetters, adjuvants, solubilizers, penetration enhancers, protective colloids, adhesion agents, thickeners, humectants, repellents, attractants, feeding stimulants, compatibilizers, bactericides, anti-freezing agents, anti-foaming agents, colorants, tackifiers and binders.

Suitable solvents and liquid carriers are water and organic solvents, such as mineral oil fractions of medium to high boiling point, e. g. kerosene, diesel oil; oils of vegetable or animal origin; aliphatic, cyclic and aromatic hydrocarbons, e. g. toluene, paraffin, tetrahydronaphthalene, alkylated naphthalenes; alcohols, e. g. ethanol, propanol, butanol, benzyl alcohol, cyclohexanol; glycols; DMSO; ketones, e. g. cyclohexanone; esters, e. g. lactates, carbonates, fatty acid esters, gamma-butyrolactone; fatty acids; phosphonates; amines; amides, e. g. N-methyl pyrrolidone, fatty acid dimethyl amides; and mixtures thereof.

Suitable solid carriers or fillers are mineral earths, e. g. silicates, silica gels, talc, kaolins, limestone, lime, chalk, clays, dolomite, diatomaceous earth, bentonite, calcium sulfate, magnesium sulfate, magnesium oxide; polysaccharides, e. g. cellulose, starch; fertilizers, e. g. ammonium sulfate, ammonium phosphate, ammonium nitrate, ureas; products of vegetable origin, e. g. cereal meal, tree bark meal, wood meal, nutshell meal, and mixtures thereof.

Suitable surfactants are surface-active compounds, such as anionic, cationic, nonionic and amphoteric surfactants, block polymers, polyelectrolytes, and mixtures thereof. Such surfactants can be used as emulsifier, dispersant, solubilizer, wetter, penetration enhancer, protective colloid, or adjuvant. Examples of surfactants are listed in McCutcheon's, Vol.1: Emulsifiers & Detergents, McCutcheon's Directories, Glen Rock, USA, 2008 (International Ed. or North American Ed.).

Suitable anionic surfactants are alkali, alkaline earth or ammonium salts of sulfonates, sulfates, phosphates, carboxylates, and mixtures thereof. Examples of sulfonates are alkylaryl sulfonates, diphenyl sulfonates, alpha-olefin sulfonates, lignin sulfonates, sulfonates of fatty acids and oils, sulfonates of ethoxylated alkylphenols, sulfonates of alkoxylated arylphenols, sulfonates of condensed naphthalenes, sulfonates of dodecyl- and tridecylbenzenes, sulfonates of naphthalenes and alkyl naphthalenes, sulfosuccinates or sulfosuccinamates. Examples of sulfates are sulfates of fatty acids and oils, of ethoxylated alkylphenols, of alcohols, of ethoxylated alcohols, or of fatty acid esters. Examples of phosphates are phosphate esters. Examples of carboxylates are alkyl carboxylates, and carboxylated alcohol or alkylphenol ethoxylates.

Suitable nonionic surfactants are alkoxylates, N-substituted fatty acid amides, amine oxides, esters, sugar-based surfactants, polymeric surfactants, and mixtures thereof. Examples of alkoxylates are compounds such as alcohols, alkylphenols, amines, amides, arylphenols, fatty acids or fatty acid esters which have been alkoxylated with 1 to 50 equivalents. Ethylene oxide and/or propylene oxide may be employed for the alkoxylation, preferably ethylene oxide. Examples of N-substituted fatty acid amides are fatty acid glucamides or fatty acid alkanolamides. Examples of esters are fatty acid esters, glycerol esters or monoglycerides. Examples of sugar-based surfactants are sorbitans, ethoxylated sorbitans, sucrose and glucose esters or alkylpolyglucosides. Examples of polymeric surfactants are home- or copolymers of vinyl pyrrolidone, vinyl alcohols, or vinyl acetate.

Suitable cationic surfactants are quaternary surfactants, for example quaternary ammonium compounds with one or two hydrophobic groups, or salts of long-chain primary amines. Suitable amphoteric surfactants are alkylbetains and imidazolines. Suitable block polymers are block polymers of the A-B or A-B-A type comprising blocks of polyethylene oxide and polypropylene oxide, or of the A-B-C type comprising alkanol, polyethylene oxide and polypropylene oxide. Suitable polyelectrolytes are polyacids or polybases. Examples of polyacids are alkali salts of polyacrylic acid or polyacid comb polymers. Examples of polybases are polyvinyl amines or polyethylene amines.

Suitable adjuvants are compounds, which have a negligible or even no pesticidal activity themselves, and which improve the biological performance of the compound I on the target. Examples are surfactants, mineral or vegetable oils, and other auxiliaries. Further examples are listed by Knowles, Adjuvants and additives, Agrow Reports DS256, T&F Informa UK, 2006, chapter 5.

Suitable thickeners are polysaccharides (e. g. xanthan gum, carboxymethyl cellulose), inorganic clays (organically modified or unmodified), polycarboxylates, and silicates.

Suitable bactericides are bronopol and isothiazolinone derivatives such as alkylisothiazolinones and benzisothiazolinones.

Suitable anti-freezing agents are ethylene glycol, propylene glycol, urea and glycerin. Suitable anti-foaming agents are silicones, long chain alcohols, and salts of fatty acids.

Suitable colorants (e. g. in red, blue, or green) are pigments of low water solubility and water-soluble dyes. Examples are inorganic colorants (e. g. iron oxide, titan oxide, iron hexacyanoferrate) and organic colorants (e. g. alizarin-, azo- and phthalocyanine colorants).

Suitable tackifiers or binders are polyvinyl pyrrolidones, polyvinyl acetates, polyvinyl alcohols, polyacrylates, biological or synthetic waxes, and cellulose ethers.

Examples for composition types and their preparation are:

### i) Water-soluble concentrates (SL, LS)

10-60 wt% of a compound I and 5-15 wt% wetting agent (e. g. alcohol alkoxylates) are dissolved in water and/or in a water-soluble solvent (e. g. alcohols) ad 100 wt%. The active substance dissolves upon dilution with water.

### ii) Dispersible concentrates (DC)

5-25 wt% of a compound I and 1-10 wt% dispersant (e. g. polyvinyl pyrrolidone) are dissolved in organic solvent (e. g. cyclohexanone) ad 100 wt%. Dilution with water gives a dispersion.

### iii) Emulsifiable concentrates (EC)

15-70 wt% of a compound I and 5-10 wt% emulsifiers (e. g. calcium dodecylbenzenesulfonate and castor oil ethoxylate) are dissolved in water-insoluble organic solvent (e. g. aromatic hydrocarbon) ad 100 wt%. Dilution with water gives an emulsion.

### iv) Emulsions (EW, EO, ES)

5-40 wt% of a compound I and 1-10 wt% emulsifiers (e. g. calcium dodecylbenzenesulfonate and castor oil ethoxylate) are dissolved in 20-40 wt% water-insoluble organic solvent (e. g. aromatic hydrocarbon). This mixture is introduced into water ad 100 wt% by means of an emulsifying machine and made into a homogeneous emulsion. Dilution with water gives an emulsion.

### v) Suspensions (SC, OD, FS)

In an agitated ball mill, 20-60 wt% of a compound I are comminuted with addition of 2-10 wt% dispersants and wetting agents (e. g. sodium lignosulfonate and alcohol ethoxylate), 0.1-2 wt% thickener (e. g. xanthan gum) and water ad 100 wt% to give a fine active substance suspension. Dilution with water gives a stable suspension of the active substance. For FS type composition up to 40 wt% binder (e. g. polyvinyl alcohol) is added.

### vi) Water-dispersible granules and water-soluble granules (WG, SG)

50-80 wt% of a compound I are ground finely with addition of dispersants and wetting agents (e. g. sodium lignosulfonate and alcohol ethoxylate) ad 100 wt% and prepared as water-dispersible or water-soluble granules by means of technical appliances (e. g. extrusion, spray tower, fluidized bed). Dilution with water gives a stable dispersion or solution of the active substance.

### vii) Water-dispersible powders and water-soluble powders (WP, SP, WS)

50-80 wt% of a compound I are ground in a rotor-stator mill with addition of 1-5 wt% dispersants (e. g. sodium lignosulfonate), 1-3 wt% wetting agents (e. g. alcohol ethoxylate) and solid carrier (e. g. silica gel) ad 100 wt%. Dilution with water gives a stable dispersion or solution of the active substance.

### viii) Gel (GW, GF)

In an agitated ball mill, 5-25 wt% of a compound I are comminuted with addition of 3-10 wt% dispersants (e. g. sodium lignosulfonate), 1-5 wt% thickener (e. g. carboxymethyl cellulose) and water ad 100 wt% to give a fine suspension of the active substance. Dilution with water gives a stable suspension of the active substance.

### ix) Microemulsion (ME)

5-20 wt% of a compound I are added to 5-30 wt% organic solvent blend (e. g. fatty acid dimethyl amide and cyclohexanone), 10-25 wt% surfactant blend (e. g. alcohol ethoxylate and arylphenol ethoxylate), and water ad 100 %. This mixture is stirred for 1 h to produce spontaneously a thermodynamically stable microemulsion.

### x) Microcapsules (CS)

An oil phase comprising 5-50 wt% of a compound I, 0-40 wt% water insoluble organic solvent (e. g. aromatic hydrocarbon), 2-15 wt% acrylic monomers (e. g. methylmethacrylate, methacrylic acid and a di- or triacrylate) are dispersed into an aqueous solution of a protective colloid (e. g. polyvinyl alcohol). Radical polymerization results in the formation of poly(meth)acrylate microcapsules. Alternatively, an oil phase comprising 5-50 wt% of a compound I according to the invention, 0-40 wt% water insoluble organic solvent (e. g. aromatic hydrocarbon), and an isocyanate monomer (e. g. diphenylmethene-4,4'-diisocyanatae) are dispersed into an aqueous solution of a protective colloid (e. g. polyvinyl alcohol). The addition of a polyamine (e. g. hexamethylenediamine) results in the formation of polyurea microcapsules. The monomers amount to 1-10 wt%. The wt% relate to the total CS composition.

### xi) Dustable powders (DP, DS)

1-10 wt% of a compound I are ground finely and mixed intimately with solid carrier (e. g. finely divided kaolin) ad 100 wt%.

### xii) Granules (GR, FG)

0.5-30 wt% of a compound I is ground finely and associated with solid carrier (e. g. silicate) ad 100 wt%. Granulation is achieved by extrusion, spray-drying or fluidized bed. xiii) Ultra-low volume liquids (UL)

1-50 wt% of a compound I are dissolved in organic solvent (e. g. aromatic hydrocarbon) ad 100 wt%.

The compositions types i) to xiii) may optionally comprise further auxiliaries, such as 0.1-1 wt% bactericides, 5-15 wt% anti-freezing agents, 0.1-1 wt% anti-foaming agents, and 0.1-1 wt% colorants.

The agrochemical compositions generally comprise between 0.01 and 95%, preferably between 0.1 and 90%, and in particular between 0.5 and 75%, by weight of active substance. The active substances are employed in a purity of from 90% to 100%, preferably from 95% to 100% (according to NMR spectrum).

For the purposes of foliar tratment, the agrochemical compositions are preferably liquid formulations composition types such as suspensions (e. g. SC, OD, FS), emulsifiable concentrates (e. g. EC) and emulsions (e. g. EW, EO, ES, ME); and comprise between 100 and 500 g compounds I per liter, even more preferably 200 to 400 g compounds I per liter.

For the purposes of treatment of plant propagation materials, particularly seeds, solutions for seed treatment (LS), Suspoemulsions (SE), flowable concentrates (FS), powders for dry treatment (DS), water-dispersible powders for slurry treatment (WS), water-soluble powders (SS), emulsions (ES), emulsifiable concentrates (EC), and gels (GF) are usually employed. The compositions in question give, after two-to-tenfold dilution, active substance concentrations of from 0.01 to 60% by weight, preferably from 0.1 to 40%, in the ready-to-use preparations. Application can be carried out before or during sowing. Methods for applying compound I and compositions thereof, respectively, onto plant propagation material, especially seeds, include dressing, coating, pelleting, dusting, and soaking as well as in-furrow application methods. Preferably, compound I or the compositions thereof, respectively, are applied on to the plant propagation material by a method such that germination is not induced, e. g. by seed dressing, pelleting, coating and dusting.

In treatment of plant propagation materials such as seeds, e. g. by dusting, coating or drenching seed, amounts of active substance of from 0.01 to 10 kg, preferably from 1 to 1000 g, more preferably from 1 to 100 g and most preferably from 5 to 100 g, per 100 kilogram of plant propagation material (preferably seeds) are generally required.

Accordingly, the present invention also relates to seed coated with compound I e.g. in an amount of from 0.1 g to 10 kg per 100 kg of seed. Accordingly, the present invention also relates to seed comprising compound I e.g. in an amount of from 0.01 g to 10 kg per 100 kg of seed. Preferalby these seed are soybean seed.

When used in the protection of materials or stored products, the amount of active substance applied depends on the kind of application area and on the desired effect. Amounts customarily applied in the protection of materials are 0.001 g to 2 kg, preferably 0.005 g to 1 kg, of active substance per cubic meter of treated material.

Various types of oils, wetters, adjuvants, fertilizer, or micronutrients may be added to the active substances or the compositions comprising them as premix or, if appropriate not until immediately prior to use (tank mix). These agents can be admixed with the compositions according to the invention in a weight ratio of 1:100 to 100:1, preferably 1:10 to 10:1.

### I. Synthesis examples

Compound I.1 has been obtained according to methods described in WO 00/0311023. With appropriate modification of the starting materials, these procedures can be used to obtain further compounds I. The resulting compounds, together with physical data, are listed in Table I below.

**Table I. Compounds of formula I with physical data (melting point [°C]; ¹H-NMR (CDCI₃) (δ); HPLC/MS retention time [min])**

| **No.** | **R** | **R¹** | **R²** | **R³** | **m.p. [°C]; ¹H-NMR (δ); Rt [min]** |
|---|---|---|---|---|---|
| I.1 | H | CH₃ | CH₃ | CH₃ | 75-80 °C |
| I.2 | H | CH₃ | CH₃ | CH₂CH₃ | 78-83 °C |
| I.3 | H | CH₃ | CH₂CH₃ | CH₃ | 79-88 °C |
| I.4 | H | CH₃ | CH₂CH₃ | CH₂CH₃ | |
| I.5 | Cl | CH₃ | CH₃ | CH₃ | (CDCl₃): δ = 1.4 (s); 1.82 (s); 1.96 (s); 2.86 (d); 3.9 (s); 5.7 (s); |
| I.6 | F | CH₃ | CH₃ | CH₃ | (CDCl₃): δ = 1.4 (s); 1.80 (s); 1.92 (s); 2.89 (d); 3.91 (s); 5.7 (s); |
| I.7 | H | CH₂CH₃ | CH₂CH₃ | CH₃ | |

### II. Comparative examples for fungicidal activity

### A. Glasshouse trials

The spray solutions were prepared in several steps:
The stock solution were prepared: a mixture of acetone and/or dimethylsulfoxide and the wetting agent/emulsifier Wettol, which is based on ethoxylated alkylphenoles, in a relation (volume) solvent-emulsifier of 99 to 1 was added to 25 mg of the compound to give a total of 5 ml.

Water was then added to total volume of 100 ml.

This stock solution was diluted with the described solvent-emulsifier-water mixture to the given concentration.

### Use example 1: Curative control of soy bean rust on soy beans caused by Phakopsora pachyrhizi

Leaves of pot-grown soybean seedlings were inoculated with spores of *Phakopsora pachyrhizi.* To ensure the success of the artificial inoculation, the plants were transferred to a humid chamber with a relative humidity of about 95 % and 20 to 24 °C for 24 h. The next day the plants were cultivated for 3 days in a greenhouse chamber at 23-27 °C and a relative humidity between 60 and 80 %. Then the plants were sprayed to run-off with an aqueous suspension, containing the concentration of active ingredient or their mixture as described below. The plants were allowed to air-dry. Then the trial plants were cultivated for 14 days in a greenhouse chamber at 23-27 °C and a relative humidity between 60 and 80 %. The extent of fungal attack on the leaves was visually assessed as % diseased leaf area. Efficacy of the fungicidal activity has been dertimed according to Abbot's formula. The results are given in the following Table.

| **Compound** | **Structure** | **Disease (%) at 4ppm** | **Efficacy (%) at 4ppm** |
|---|---|---|---|
| 1.1 (according to the invention) | 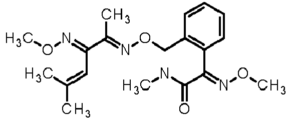 | 8 | 90 |
| Trifloxystrobin (Comparative example) | 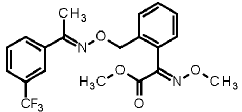 | 45 | 43.8 |
| Pyraclostrobin (Comparative example) | 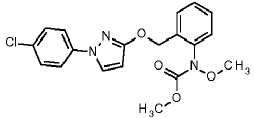 | 65 | 18.8 |
| Azoxystrobin (Comparative example) | 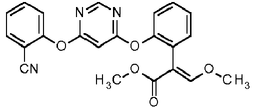 | 55 | 31.3 |
| Untreated control | | 80 | 0 |

## Claims

1. Use of compounds of formula I 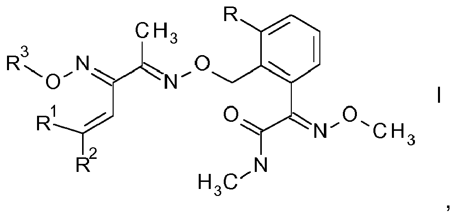 wherein
R is hydrogen, halogen or methyl;
R¹,R² independently of each other are selected from hydrogen, C₁-C₄-alkyl and C₁-C₄-haloalkyl;
or R¹ and R² together with the carbon atom linking them form a C₃-C₆-cycloalkyl;
R³ is C₁-C₄-alkyl;
for combating soybean rust.

2. The use according to claim 1 of the compounds of formula I, wherein R is hydrogen.

3. The use according to any one of the claims 1 to 2 of the compounds of formula I, wherein R¹ and R² are both C₁-C₄-alkyl.

4. The use according to any one of the claims 1 to 3 of the compounds of formula I, wherein R³ is methyl.

5. The use according to claim 4 of the compound of formula 1.1

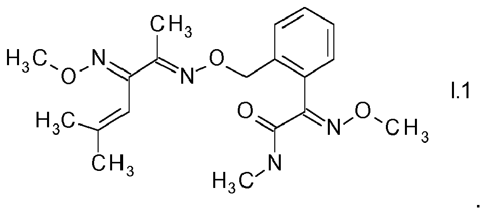

6. The use according to any one of the claims 1 to 5 for combating *Phakopsora pachyrhizi* and/or *P*. *meibomiae* (soybean rust) on soybean plants.

7. The use according to any one of the claims 1 to 6 for combating *Phakopsora pachyrhizi* and/or *P*. *meibomiae* (soybean rust) and at least one additional soybean pathogen selected from *Cercospora sojina* and *C. kikuchii, Colleotrichum gloeosporioides, Corynespora cassiicola* (leaf spots), *Dematophora* (teleomorph: *Rosellinia*) *necatrix* (root and stem rot), *Diaporthe* spp., e. g. *D. phaseolorum* (damping off), *Fusarium solani, Microsphaera diffusa* (powdery mildew), *Peronospora manshurica* (downy mildew), *Phytophthora megasperma* (syn. *P. sojae), Rhizoctonia solani* (root and stem rot), *Septoria glycines* (brown spot) and *Thielaviopsis* spp. (black root rot).

8. A method for combating soybean rust, comprising:
treating the soybean plants or soybean plant propagation material to be protected against attack by *Phakopsora pachyrhizi* and/or *P*. *meibomiae* with an effective amount of at least one compound of formula I as defined in any of the claims 1 to 5, or a composition comprising such compound of formula I as defined in any of the claims 1 to 5.

9. Use of compound of formula I as defined in any of the claims 1 to 5 for improving the health of a soybean plant.

10. Seed coated with compound of formula I as defined in any of the claims 1 to 5 in an amount of from 0.01 g to 10 kg per 100 kg of seed.

11. Compounds of formula I 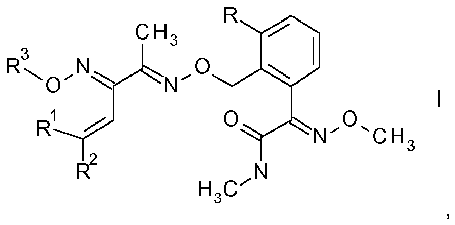 wherein
R is hydrogen, halogen or methyl;
R¹,R² independently of each other are selected from hydrogen, C₁-C₄-alkyl and C₁-C₄-haloalkyl;
or R¹ and R² together with the carbon atom linking them form a C₃-C₆-cycloalkyl;
R³ is C₁-C₄-alkyl;
except for (2*E*)-2-methoxyimino-2-[2-[[(*E*)-[(2*E*)-2-methoxyimino-1,4-dimethyl-pent-3-en-ylidene]amino]oxymethyl]phenyl]-*N*-methyl-acetamide.

12. Agrochemical compositions wherein said compositions comprise an auxiliary and at least one compound of formula I as defined in claim 11.
